# EUROPEAN PATENT SPECIFICATION

(11) **EP 3 226 087 B1**
(45) Date of publication and mention of the grant of the patent: **09.12.2026**
(21) Application number: 15791468.0
(22) Date of filing: 17.04.2015
(51) Int. Cl.: G05B 19/04, G08B 21/00, A61B 5/00

(54) **ELECTRONIC DEVICE AND CONTROL METHOD THEREFOR**
ELEKTRONISCHE VORRICHTUNG UND STEUERUNGSVERFAHREN DAFÜR
DISPOSITIF ÉLECTRONIQUE ET PROCÉDÉ DE COMMANDE ASSOCIÉ

(30) Priority: 28.11.2014 CN 201410710653
(43) Date of publication of application: 04.10.2017
(73) Proprietor: BOE Technology Group Co., Ltd., Beijing 100015 (CN)
(72) Inventor: MA, Zhenfu, Beijing 100176 (CN)
(74) Representative: Cohausz & Florack
(86) International application number: PCT/CN2015/076849
(87) International publication number: WO 2016/082435

(56) References cited:
- EP-A2- 2 058 162
- WO-A1-2014/057921
- CN-A- 1 653 787
- CN-A- 103 860 148
- CN-A- 104 104 983
- CN-A- 104 503 281
- CN-U- 204 314 640
- JP-A- 2014 175 839
- US-A1- 2004 116 784
- US-A1- 2014 276 165
- US-A1- 2014 281 971

## Description

### FIELD OF THE INVENTION

The present invention relates to the field of control of electronic devices, and specifically relates to a control method of an electronic device and an electronic device capable of executing the control method.

### BACKGROUND OF THE INVENTION

At present, mobile terminal devices, such as mobile phones, tablet computers and the like, have become indispensable tools in people's daily life, especially with the increasing intellectualization of such mobile terminal devices, people use these mobile terminal devices more frequently. Many people are used to using these mobile terminal devices before bedtime. However, a user, who forgets to turn off these mobile terminal devices or to set these mobile terminal devices into the flight mode, will be exposed to radiation from communication signals, and the waste of electrical energy will be caused.

With the continuous improvement of living standards, people pay more and more attention to personal health. At present, there have been many commercially available wearable devices already provided with a function of detecting user's health. However, the existing wearable device is low in the level of integration. In order to obtain a health detection function, a user needs to purchase individual wearable devices. As a result, the user needs to wear an additional device, which is inconvenient in daily life and increases the user's expense as well.

Therefore, how to reduce the impact of communication signals on human body has become a technical problem to be solved urgently. WO 2014/057921 A1 discloses a control method of an electronic device, comprising acquiring real-time physiological information of a user, judging whether the real-time physiological information of the user is within a predetermined range, and selectively adjusting an operating mode of the electronic device to a predetermined mode if the real-time physiological information of the user is within the predetermined range, which includes controlling the electronic device to display prompt information in order to remind the user whether to adjust the operating mode of the electronic device to the predetermined mode.

### SUMMARY OF THE INVENTION

An objective of the present invention is to provide a control method of an electronic device and an electronic device, and by controlling the electronic device with the control method, the exposure of the human body to radiation from the electronic device can be reduced. The present invention is directed to a control method of an electronic device as defined in independent claim 1 and a corresponding electronic device as defined in independent claim 3. Further aspects of the invention are defined in dependent claims.

According to one aspect, there is provided a control method of an electronic device, including steps of: acquiring a real-time breathing rate of a user; judging whether the real-time breathing rate of the user is within a predetermined range; and selectively adjusting an operating mode of the electronic device to a predetermined mode if the real-time breathing rate of the user is within the predetermined range.

According to embodiments, the predetermined mode may be a distraction free mode.

According to the embodiments, the step of selectively adjusting the operating mode of the electronic device to the predetermined mode may include: controlling the electronic device to display prompt information in order to remind the user whether to adjust the operating mode of the electronic device to the predetermined mode, and controlling the electronic device to send a vibration signal; and adjusting the operating mode of the electronic device to the predetermined mode, after the user selects to adjust the operating mode of the electronic device to the predetermined mode according to the prompt information.

According to the embodiments, the step of selectively adjusting the operating mode of the electronic device to the predetermined mode may further include: adjusting the operating mode of the electronic device to the predetermined mode, if the electronic device fails to receive a selection instruction from the user after a predetermined period of time has passed since the electronic device displays the prompt information.

According to the embodiments, the operating mode of the electronic device may include a disease warning mode, and in the disease warning mode, the control method may further include a step of: controlling the electronic device to send out a distress signal, if the real-time breathing rate of the user is higher than an upper limit of the predetermined range by a first predetermined value, or if the real-time breathing rate of the user is lower than a lower limit of the predetermined range by a second predetermined value.

According to the embodiments, a way in which the electronic device sends out the distress signal may include calling a preset phone number.

According to the embodiments, the operating mode of the electronic device may include a health checkup mode, and in the health checkup mode, the control method may further include steps of: determining whether the user is healthy according to the breathing rate of the user; and controlling the electronic device to display information for reminding the user of a health checkup, if the user is determined to be unhealthy.

According to the embodiments, the step of determining whether the user is healthy according to the breathing rate of the user may include: comparing the acquired breathing rate of the user with a healthy breathing rate range, and determining that the user is unhealthy if the real-time breathing rate of the user goes beyond the healthy breathing rate range; and/or recording the breathing rate of the user and sending it to a remote diagnosis platform, and judging, by the remote diagnosis platform, whether the user is healthy.

According to the embodiments, the step of acquiring the real-time breathing rate of the user may include: acquiring a real-time breathing audio of the user; and acquiring the real-time breathing rate of the user according to the acquired real-time breathing audio.

According to another aspect, there is provided an electronic device, including: a real-time breathing rate acquisition module, configured to acquire a real-time breathing rate of a user; a comparison module, configured to compare the real-time breathing rate of the user with an upper limit value and a lower limit value of a predetermined range, and to generate a result of comparison; and an operating mode adjusting module, configured to acquire the result of comparison generated by the comparison module, and to selectively adjust an operating mode of the electronic device to a predetermined mode if the real-time breathing rate of the user is within the predetermined range.

According to embodiments, the predetermined mode may include a distraction free mode.

According to the embodiments of the present invention, the electronic device may further include a prompt information generation module, which can generate prompt information reminding the user whether to adjust the operating mode of the electronic device to the predetermined mode, if the real-time breathing rate of the user is within the predetermined range. The electronic device may display the prompt information generated by the prompt information generation module, and meanwhile generate a vibration signal, and the operating mode adjusting module may adjust the operating mode of the electronic device to the predetermined mode, after the user selects to adjust the operating mode of the electronic device to the determined mode according to the prompt information.

According to the embodiments, the operating mode adjusting module may adjust the operating mode of the electronic device to the predetermined mode, if the electronic device fails to receive a selection instruction from the user after a predetermined period of time has passed since the electronic device displays the prompt information.

According to the embodiment,s the electronic device may further include a distress signal sending module, which sends out a distress signal, if the real-time breathing rate of the user is higher than an upper limit of the predetermined range by a first predetermined value, or if the real-time breathing rate of the user is lower than a lower limit of the predetermined range by a second predetermined value.

According to the embodiments, the electronic device may further include a communication module, and a way in which the distress signal sending module sends out the distress signal includes calling a preset phone number by using the communication module.

According to the embodiments, the comparison module may be further configured to determine whether a user is healthy according to the breathing rate of the user, and if the user is determined to be unhealthy, the comparison module generates health checkup prompt information for reminding the user of a health checkup, and the electronic device stores and displays the health checkup prompt information.

According to the embodiments, the electronic device may further include a data storage module storing a healthy breathing rate database. The comparison module may compare the acquired breathing rate of the user with healthy breathing rates in the database, and determine that the user is unhealthy if the breathing rate of the user goes beyond a range of the healthy breathing rates.

According to the embodiments, the electronic device may further include a communication module, configured to send a record of the breathing rate of the user to a remote diagnosis platform. The remote diagnosis platform may judge whether the user is healthy, and the electronic device receives and displays a result of judgment sent from the remote diagnosis platform.

According to the embodiments, the electronic device may further include: a user-defined module, configured to store a predetermined range corresponding to the breathing rate of the user; and/or a range generation module, configured to calculate an average breathing rate range of the user, as the predetermined range, according to the real-time breathing rates of the user acquired by the real-time breathing rate acquisition module in a predetermined period of time.

According to the embodiments, the electronic device may further include an audio collection module, configured to collect a real-time breathing audio of the user. The real-time breathing rate acquisition module may acquire the real-time breathing rate of the user according to the real-time breathing audio of the user collected by the audio collection module.

According to the embodiments, the electronic device may further include a breathing sensor, configured to sense real-time breathing of the user. The real-time breathing rate acquisition module may acquire the real-time breathing rate of the user according to the real-time breathing of the user sensed by the breathing sensor.

According to the concept of the present disclosure, it is determined that the user has fallen asleep if the acquired real-time breathing rate of the user is within a predetermined range indicative of the sleep state of the user. In this case, the operating mode of the electronic device can be adjusted to the predetermined mode, for example, a distraction free mode including a flight mode. After the electronic device is adjusted to the distraction free mode, the exposure of the human body to radiation of signals from the electronic device can be reduced, and the power consumption of the electronic device can be decreased.

### BRIEF DESCRIPTION OF THE DRAWINGS

The accompany drawings are provided for further understanding the present invention, and constitute a part of the specification. They are used for explaining the present invention together with the following specific implementations, but not intended to limit the present invention. In which:
Fig. 1 is a flowchart of a control method of an electronic device according to embodiments of the present invention;
Fig. 2 is a flowchart of a control method in a disease warning mode according to the embodiments of the present invention;
Fig. 3 is a flowchart of a control method in a health checkup mode according to the embodiments of the present invention;
Fig. 4 shows a specific implementation of the step of determining whether a user is healthy according to a breathing rate of the user shown in Fig. 3; and
Fig. 5 is a schematic block diagram of an electronic device according to the embodiments of the present invention.

### DESCRIPTION OF THE PREFERRED EMBODIMENTS

The specific implementations of the present invention will be described in detail below with reference to the accompany drawings. It should be understood that the specific implementations described herein are merely used for describing and explaining the present invention, but not for limiting the present invention.

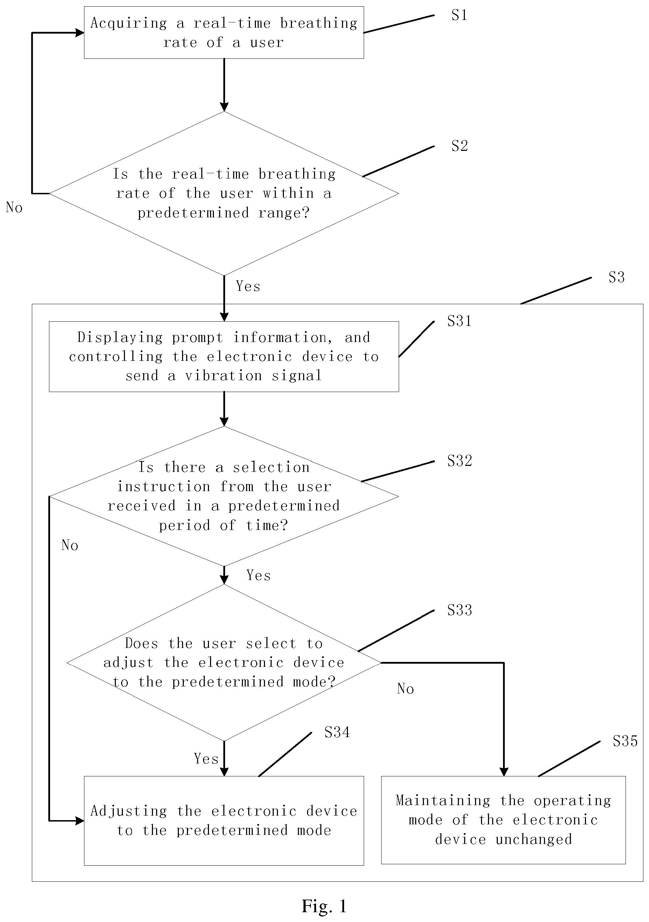

Fig. 1 is a flowchart of a control method of an electronic device according to embodiments of the present invention.

Referring to Fig. 1, the control method of an electronic device according to embodiments of the present invention includes steps of:
acquiring a real-time breathing rate of a user (step S1);
judging whether the real-time breathing rate of the user is within a predetermined range (step S2); and
selectively adjusting an operating mode of the electronic device to a predetermined mode if the real-time breathing rate of the user is within the predetermined range (step S3).

Herein, "user" refers to a user who uses the electronic device. The predetermined mode may be (but not limited to), for example, a distraction free mode including a flight mode. According to the embodiments of the present invention, "distraction free mode" may include any one or more of an OFF mode, a silent mode and a flight mode. Additionally, the user may self-define the distraction free mode.

By controlling the electronic device by the control method according to the embodiments of the present invention, after adjusting the electronic device to the distraction free mode, the exposure of the human body to radiation of signals from the electronic device can be reduced, and the power consumption of the electronic device can be decreased.

The breathing rate of a normal person in the sleep state ranges from 16 breaths per minute to 20 breaths per minute. Therefore, the predetermined range may be set to be from 16 breaths per minute to 20 breaths per minute. If the breathing rate of the user is judged to be within the predetermined range, it is indicated that the user has fallen into the sleep state. In this case, the operating mode of the electronic device may be selectively adjusted to the predetermined mode (for example, the flight mode).
"selectively adjusting the operating mode of the electronic device to the predetermined mode" herein may include at least two situations: first, the operating mode of the electronic device is directly adjusted to the predetermined mode; second, the operating mode of the electronic device is adjusted to the predetermined mode at the user's will.

When the user is at rest, it is possible that his/her breathing rate is within the predetermined range. Therefore, it is inappropriate to directly adjust the operating mode of the electronic device to the predetermined mode as in the first situation, and it is more appropriate to employ the processing way as in the second situation.

In the second situation, the electronic device may be controlled to display prompt information to remind the user whether to adjust the operating mode of the electronic device to the predetermined mode, and the electronic device is controlled to send a vibration signal (step S31). After the user selects to adjust the operating mode of the electronic device to the predetermined mode according to the prompt information ("YES" in the step S33), the operating mode of the electronic device is adjusted to the predetermined mode (step S34).

If the user is at rest (but has not fallen asleep yet), and "NO" is selected in the step S33, that is, the user does not want to adjust the operating mode of the electronic device, and thus the operating mode of the electronic device is maintained unchanged ( step S35).

Additionally, if the electronic device fails to receive a selection instruction from the user after a predetermined period of time has passed since the electronic device displays the prompt information ("NO" in the step S32), the operating mode of the electronic device may also be adjusted to the predetermined mode (step S34).

According to the embodiments of the present invention, the predetermined period of time may be (but not limit to), for example, 10s, 20s and so on. However, the predetermined period of time should not be too long, for example, the predetermined period of time should not exceed 1 min.

According to the embodiments of the present invention, the real-time breathing rate may be obtained in the following ways. For example, a real-time breathing audio may be obtained by recording, and the number of breaths of the user in the breathing audio in a predetermined period of time is monitored, thus to obtain the real-time breathing rate of the user. According to an embodiment of the present invention, the step S1 may include:
acquiring a real-time breathing audio of the user; and
acquiring the real-time breathing rate of the user according to the acquired real-time breathing audio.

According to another embodiment of the present invention, the breathing rate of the user may be obtained by, for example, a breathing sensor.

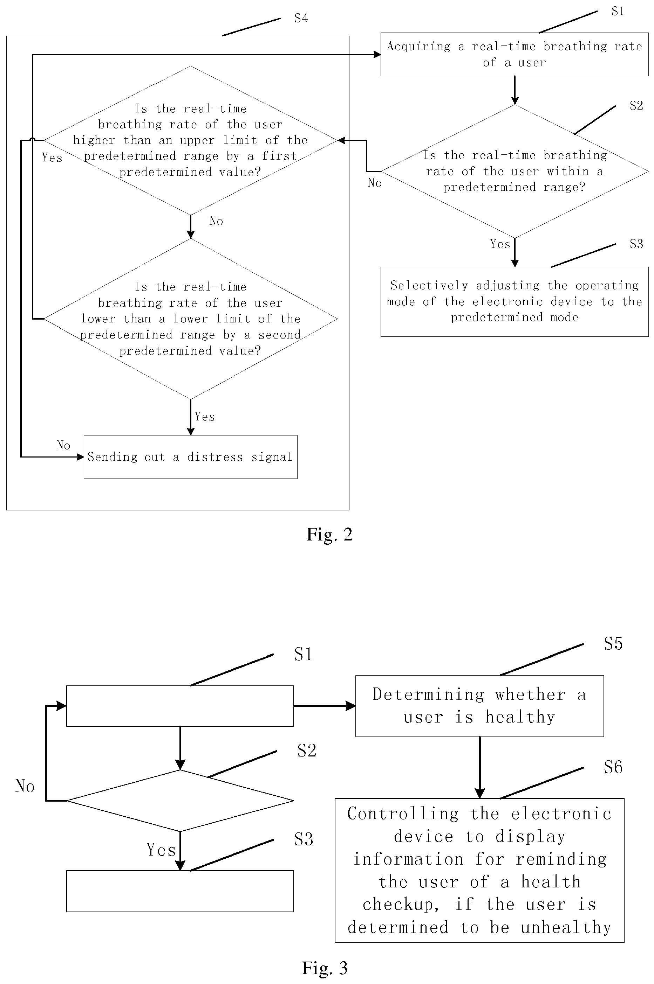

If the breathing rate of the user is not within the predetermined range, it is indicated that the user may be awake. Additionally, if the breathing rate of the user goes beyond the predetermined range again after the user has been determined to be in the sleep state, the user may be in an abnormal sleep state. According to the embodiments of the present invention, the operating mode of the electronic device includes a disease warning mode. Fig. 2 is a flowchart of a control method in a disease warning mode according to the embodiments of the present invention. Referring to Fig. 2, in the disease warning mode, the control method may further include a step of:
controlling the electronic device to send out a distress signal, if the real-time breathing rate of the user is higher than an upper limit of the predetermined range by a first predetermined value, or if the real-time breathing rate of the user is lower than a lower limit of the predetermined range by a second predetermined value (step S4).

The abnormal sleep state may mean that some symptoms appear when the user is in the sleep state. For example, if the real-time breathing rate of the user is higher than the upper limit of the predetermined range by the first predetermined value, it is indicated that the user probably has symptoms such as fever, pain, anemia, hyperthyroidism, heart failure or the like. If the real-time breathing rate of the user is lower than the lower limit of the predetermined range by the second predetermined value, it is indicated that the intracranial pressure of the user is increased, which is very dangerous as well. For the life safety of the user, an alarm should be sent out in time, when it is monitored that the real-time breathing rate of the user goes beyond the predetermined range by a specific predetermined value.

According to the embodiments of the present invention, a way in which the electronic device sends out the distress signal may include (but not limit to), for example, calling a preset phone number.

The preset phone number may be set by the user. For example, the preset phone number may be a phone number of a family member of the user, or may be a phone number of a doctor in charge of the user. Additionally, the preset phone number may be a phone number of an emergency center.

According to the embodiments of the present invention, the way in which the electronic device sends out the distress signal may further include sending out an audible signal or sending out the distress signal over a network.

According to the embodiments of the present invention, the first predetermined value and the second predetermined value may be set by the user according to his/her situations, or set for the user by the doctor in charge of the user.

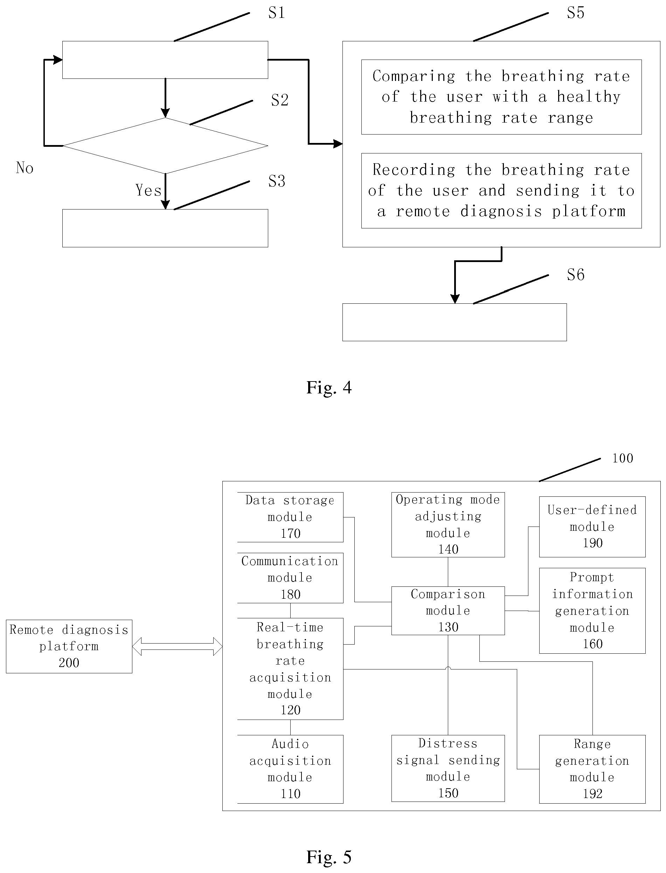

According to the embodiments of the present invention, the operating mode of the electronic device includes a health checkup mode. Fig. 3 is a flowchart of a control method in a health checkup mode according to the embodiments of the present invention, and Fig. 4 shows a specific implementation of the step of determining whether a user is healthy according to a breathing rate of the user shown in Fig. 3.

Referring to Fig. 3, in the health checkup mode, the control method may further include steps of:
determining whether a user is healthy according to the breathing rate of the user (step S5); and
controlling the electronic device to display information for reminding the user of a health checkup, if the user is determined to be unhealthy (step S6).

In the step S5, the breathing rate of the user in each time period during sleeping may be recorded, and then it is judged whether the user is healthy. The specific breathing rate, accompanied with specific changes of the breathing depth, may reflect a specific state of health, for example, respiratory muscle paralysis, serious meteorism, ascites, obesity and lung diseases (such as pneumonia, pleurisy, pleural effusion, pneumothorax and the like). With the recorded changes in the breathing rate and breathing depth of the user, the state of health of the user can be determined. The user may get a physical examination in hospital upon receipt of the information about the health checkup provided in the step S6, which is conducive to safeguard the physical health of the user.

In the present invention, t whether a user is healthy may be determined in may ways. For example, as show in Fig. 4, the step S5 may include:
comparing the acquired breathing rate of the user with a healthy breathing rate range, and determining that the user is unhealthy if the real-time breathing rate of the user goes beyond the healthy breathing rate range; and/or
recording the breathing rate of the user and sending it to a remote diagnosis platform, and judging, by the remote diagnosis platform, whether the user is healthy.

It may be easily understood that the record of the breathing rate of the user may include various extreme breathing rates of the user when he/she is in the sleep state.

After the record of the breathing rate of the user is sent to the remote diagnosis platform, a professional doctor may analyze the breathing rate, and determine whether the user is healthy and what disease the user may probably suffer from.

Fig. 5 is a schematic block diagram of an electronic device according to the embodiments of the present invention. Referring to Fig. 1 and Fig. 5, the electronic device 100 according to the embodiments of the present invention may include a real-time breathing rate acquisition module 120 for executing the step S1, a comparison module 130 for executing the step S2 and an operating mode adjusting module 140 for executing the step S3. The real-time breathing rate acquisition module 120 is configured to acquire a real-time breathing rate of a user. The comparison module 130 is configured to compare the real-time breathing rate of the user with an upper limit value and a lower limit value of the predetermined range, and to generate a result of comparison. The operating mode adjusting module 140 is configured to acquire the result of comparison generated by the comparison module 130, and to selectively adjust the operating mode of the electronic device 100 to a predetermined mode if the real-time breathing rate of the user is within the predetermined range.

According to the embodiments of the present invention, the electronic device 100 may be a mobile phone, a tablet computer, an e-book and other devices.

According to an embodiment of the present invention, the electronic device 100 may further include an audio acquisition module 110. The audio acquisition module 110 is configured to collect a real-time breathing audio of the user. The real-time breathing rate acquisition module 120 may acquire the real-time breathing rate of the user according to the real-time breathing audio of the user collected by the audio collection module 110. According to another embodiment of the present invention, the electronic device 100 may include a breathing sensor (not shown) configured to sense the real-time breathing of the user. The real-time breathing rate acquisition module 120 may acquire the real-time breathing rate of the user according to the real-time breathing of the user sensed by the breathing sensor.

According to the embodiments of the present invention, the electronic device 100 may further include a prompt information generation module 160. The prompt information generation module 160 can generate prompt information reminding the user whether to adjust the operating mode of the electronic device 100 to the predetermined mode, when the real-time breathing rate of the user is within the predetermined range. At this moment, the electronic device 100 can display the prompt information, and meanwhile, generate a vibration signal. The operating mode adjustment module 140 may adjust the operating mode of the electronic device 100 to the predetermined mode, after the user selects to adjust the operating mode of the electronic device 100 to the predetermined mode according to the prompt information.

According to the embodiments of the present invention, the operating mode adjustment mode 140 may also adjust the operating mode of the electronic device 100 to the predetermined mode, if the electronic device 100 fails to receive a selection instruction from the user after a predetermined period of time has passed since the electronic device 100 displays the prompt information. For example, when the user has fallen asleep, a selection instruction from the user cannot be received after the prompt information is displayed, and therefore, after a predetermined period of time, the operating mode of the electronic device 100 may be adjusted to the predetermined mode. According to the embodiments of the present invention, the predetermined period of time may be (but not limit to), for example, 10s, 20s and so on. However, the predetermined period of time should not be too long, for example, the predetermined period of time should not exceed 1 min.

According to the embodiments of the present invention, the operating mode of the electronic device 100 may include a disease warning mode. Correspondingly, the electronic device 100 may further include a distress signal sending module 150. The distress signal sending module 150 may send out a distress signal, when the real-time breathing rate of the user is higher than an upper limit of the predetermined range by a first predetermined value, or when the real-time breathing rate of the user is lower than a lower limit of the predetermined range by a second predetermined value.

As described above, the distress signal may be in various forms. According to an embodiment of the present invention, the electronic device 100 may include a communication module 180 (for example, the electronic device 100 is a communication device such as a mobile phone or the like). In this case, a way in which the distress signal sending module 150 sends out the distress signal may include calling a preset phone number by using the communication module 180. According to another embodiment of the present invention, the electronic device 100 may not include the communication module 180. In this case, the way in which the distress signal sending module 150 sends out the distress signal may include sending an audible signal or sending the distress signal over a network. Of course, in the case where the electronic device 100 includes the communication module 180, the distress signal may also be sent by sending out an audible signal, or over a network.

According to the embodiments of the present invention, the operating mode of the electronic device 100 may include a health checkup mode. Correspondingly, the comparison module 130 may be further configured to determine whether the user is healthy according to the breathing rate of the user. If the user is determined to be unhealthy, the comparison module 130 may generate health checkup prompt information for reminding the user of a health checkup, and the electronic device 100 may store the health checkup prompt information in a data storage module 170 and display the health checkup prompt information.

Additionally, the data storage module 170 may further store a healthy breathing rate database. The comparison module 130 may compare the acquired breathing rate of the user with the healthy breathing rates in the database, and it is determined that the user is unhealthy if the breathing rate of the user goes beyond the range of the healthy breathing rates.

Additionally, the communication module 180 may be configured to send a record of the breathing rate of the user, stored in the data storage module 170, to a remote diagnosis platform 200. The remote diagnosis platform 200 may judge whether the user is healthy, and the electronic device 100 may receive and display a result of judgment sent from the remote diagnosis platform 200.

A professional doctor may judge the state of health of the user according to the record of the breathing rates of the user uploaded to the remote diagnosis platform 200 and provide a result of treatment. The result is sent to the electronic device 100 through the remote diagnosis platform 200, and the electronic device 100 may display the result received from the remote diagnosis platform 200. The communication module 180 may be configured to receive the result sent from the remote diagnosis platform 200.

According to the embodiments of the present invention, the electronic device 100 may further include a user-defined module 191, for storing a predetermined range corresponding to the breathing rates of the user. Additionally, the electronic device 100 may further include a range generation module 192. The range generation module 192 may calculate an average breathing rate range of the user, as the predetermined range, according to the real-time breathing rates of the user acquired by the real-time breathing rate acquisition module 120 in a predetermined period of time.

It could be understood that the aforementioned implementations are exemplary implementations merely used for describing the principle of the present invention, and the present invention is not limited thereto. For a person of ordinary skill in the art, various variations and improvements may be made.

## Claims

1. A control method of an electronic device, comprising steps of:
acquiring a real-time breathing rate of a user;
judging whether the real-time breathing rate of the user is within a predetermined range; and
selectively adjusting an operating mode of the electronic device to a predetermined mode if the real-time breathing rate of the user is within the predetermined range,
wherein the predetermined mode comprises a distraction free mode in which an exposure of the user to radiation of signals from the electronic device is reduced, and
wherein the step of selectively adjusting the operating mode of the electronic device to the predetermined mode comprises:
controlling the electronic device to display prompt information in order to remind the user whether to adjust the operating mode of the electronic device to the predetermined mode, and controlling the electronic device to send a vibration signal;
adjusting the operating mode of the electronic device to the predetermined mode, after the user selects to adjust the operating mode of the electronic device to the predetermined mode according to the prompt information;
maintaining the operating mode of the electronic device unchanged, after the user selects not to adjust the operating mode of the electronic device to the predetermined mode according to the prompt information; and
adjusting the operating mode of the electronic device to the predetermined mode, if the electronic device fails to receive a selection instruction from the user after a predetermined period of time has passed since the electronic device displays the prompt information.

2. The control method according to claim 1, wherein the step of acquiring the real-time breathing rate of the user comprises:
acquiring a real-time breathing audio of the user; and
acquiring the real-time breathing rate of the user according to the acquired real-time breathing audio.

3. An electronic device, comprising:
a real-time breathing rate acquisition module, configured to acquire a real-time breathing rate of a user;
a comparison module, configured to compare the real-time breathing rate of the user with an upper limit value and a lower limit value of a predetermined range, and to generate a result of comparison;
an operating mode adjusting module, configured to acquire the result of comparison generated by the comparison module, and to selectively adjust an operating mode of the electronic device to a predetermined mode if the real-time breathing rate of the user is within the predetermined range; and
a prompt information generation module, which is capable of generating prompt information reminding the user whether to adjust the operating mode of the electronic device to the predetermined mode, if the real-time breathing rate of the user is within the predetermined range,
wherein the predetermined mode comprises a distraction free mode in which an exposure of the user to radiation of signals from the electronic device is reduced, and
wherein the electronic device displays the prompt information generated by the prompt information generation module, and meanwhile generates a vibration signal,
the operating mode adjusting module adjusts the operating mode of the electronic device to the predetermined mode, after the user selects to adjust the operating mode of the electronic device to the predetermined mode according to the prompt information,
the operating mode adjusting module maintains the operating mode of the electronic device unchanged, after the user selects not to adjust the operating mode of the electronic device to the predetermined mode according to the prompt information, and
the operating mode adjusting module adjusts the operating mode of the electronic device to the predetermined mode, if the electronic device fails to receive a selection instruction from the user after a predetermined period of time has passed since the electronic device displays the prompt information.

4. The electronic device according to claim 3, further comprising:
a distress signal sending module, which sends out a distress signal if the real-time breathing rate of the user is higher than an upper limit of the predetermined range by a first predetermined value, or if the real-time breathing rate of the user is lower than a lower limit of the predetermined range by a second predetermined value.

5. The electronic device according to claim 3, wherein the comparison module is further configured to determine whether a user is healthy according to the breathing rate of the user, and if the user is determined to be unhealthy, the comparison module generates health checkup prompt information for reminding the user of a health checkup, and the electronic device stores and displays the health checkup prompt information.

6. The electronic device according to claim 5, further comprising:
a data storage module, which stores a healthy breathing rate database,
wherein the comparison module compares the acquired breathing rate of the user with healthy breathing rates in the database, and determines that the user is unhealthy if the breathing rate of the user goes beyond a range of the healthy breathing rates, and/or
the electronic device further comprising:
a communication module, configured to send a record of the breathing rate of the user to a remote diagnosis platform,
wherein the remote diagnosis platform judges whether the user is healthy, and the electronic device receives and displays a result of judgment sent from the remote diagnosis platform.

7. The electronic device according to claim 3, further comprising:
a user-defined module, configured to store a predetermined range corresponding to the breathing rate of the user; and/or
a range generation module, configured to calculate an average breathing rate range of the user, as the predetermined range, according to the real-time breathing rates of the user acquired by the real-time breathing rate acquisition module in a predetermined period of time.

8. The electronic device according to claim 3, further comprising:
an audio collection module, configured to collect a real-time breathing audio of the user,
wherein the real-time breathing rate acquisition module acquires the real-time breathing rate of the user according to the real-time breathing audio of the user collected by the audio collection module.

9. The electronic device according to claim 3, further comprising:
a breathing sensor, configured to sense real-time breathing of the user,
wherein the real-time breathing rate acquisition module acquires the real-time breathing rate of the user according to the real-time breathing of the user sensed by the breathing sensor.

## Patentansprüche

1. Verfahren zur Steuerung einer elektronischen Vorrichtung, folgende Schritte umfassend:
Erfassen einer Echtzeit-Atemfrequenz eines Benutzers;
Beurteilen, ob die Echtzeit-Atemfrequenz des Benutzers innerhalb eines vorgegebenen Bereichs liegt; und
selektives Einstellen eines Betriebsmodus der elektronischen Vorrichtung auf einen vorgegebenen Modus, wenn die Echtzeit-Atemfrequenz des Benutzers innerhalb des vorgegebenen Bereichs liegt,
wobei der vorgegebene Modus einen störungslosen Modus umfasst, in dem eine Strahlungsbelastung des Benutzers durch von der elektronischen Vorrichtung ausgesendete Signale verringert wird, und
wobei der Schritt des selektiven Einstellens des Betriebsmodus der elektronischen Vorrichtung auf den vorgegebenen Modus umfasst:
Steuern der elektronischen Vorrichtung, um Hinweisinformationen anzuzeigen, um den Benutzer daran zu erinnern, den Betriebsmodus der elektronischen Vorrichtung auf den vorgegebenen Modus einzustellen, und Steuern der elektronischen Vorrichtung, um ein Vibrationssignal zu senden;
Einstellen des Betriebsmodus der elektronischen Vorrichtung auf den vorgegebenen Modus, nachdem der Benutzer in Abhängigkeit von den Hinweisinformationen ausgewählt hat, den Betriebsmodus der elektronischen Vorrichtung auf den vorgegebenen Modus einzustellen;
unverändertes Aufrechterhalten des Betriebsmodus der elektronischen Vorrichtung, nachdem der Benutzer in Abhängigkeit von den Hinweisinformationen ausgewählt hat, den Betriebsmodus der elektronischen Vorrichtung nicht auf den vorgegebenen Modus einzustellen; und
Einstellen des Betriebsmodus der elektronischen Vorrichtung auf den vorgegebenen Modus, falls die elektronische Vorrichtung nach Ablauf einer vorgegebenen Zeitspanne, seitdem die elektronische Vorrichtung die Hinweisinformationen angezeigt hat, keine Auswahlanweisung von dem Benutzer empfängt.

2. Verfahren zur Steuerung nach Anspruch 1, wobei der Schritt des Erfassens der Echtzeit-Atemfrequenz des Benutzers aufweist:
Erfassen eines Echtzeit-Atemgeräusches des Benutzers; und
Erfassen der Echtzeit-Atemfrequenz des Benutzers in Abhängigkeit von dem erfassten Echtzeit-Atemgeräusch.

3. Elektronische Vorrichtung, aufweisend:
ein Modul zum Erfassen der Echtzeit-Atemfrequenz, das konfiguriert ist, um eine Echtzeit-Atemfrequenz eines Benutzers zu erfassen;
ein Vergleichsmodul, das konfiguriert ist, um die Echtzeit-Atemfrequenz des Benutzers mit einem oberen Grenzwert und einem unteren Grenzwert eines vorgegebenen Bereichs zu vergleichen und ein Vergleichsergebnis zu erzeugen;
ein Betriebsmodus-Einstellungsmodul, das konfiguriert ist, um das von dem Vergleichsmodul erzeugte Vergleichsergebnis zu erfassen und einen Betriebsmodus der elektronischen Vorrichtung selektiv auf einen vorgegebenen Modus einzustellen, wenn die Echtzeit-Atemfrequenz des Benutzers innerhalb des vorgegebenen Bereichs liegt; und
ein Modul zur Erzeugung von Hinweisinformationen, das in der Lage ist, Hinweisinformationen zu erzeugen, die den Benutzer daran erinnern, ob der Betriebsmodus der elektronischen Vorrichtung auf den vorgegebenen Modus einzustellen ist, falls die Echtzeit-Atemfrequenz des Benutzers innerhalb des vorgegebenen Bereichs liegt,
wobei der vorgegebene Modus einen störungslosen Modus umfasst, in dem eine Strahlungsbelastung des Benutzers durch von der elektronischen Vorrichtung ausgesendete Signale verringert wird, und
wobei die elektronische Vorrichtung die von dem Modul zur Erzeugung von Hinweisinformationen erzeugte Hinweisinformationen anzeigt und währenddessen ein Vibrationssignal erzeugt,
das Betriebsmodus-Einstellungsmodul den Betriebsmodus der elektronischen Vorrichtung auf den vorgegebenen Modus einstellt, nachdem der Benutzer in Abhängigkeit von den Hinweisinformationen ausgewählt hat, den Betriebsmodus der elektronischen Vorrichtung auf den vorgegebenen Modus einzustellen,
das Betriebsmodus-Einstellungsmodul den Betriebsmodus der elektronischen Vorrichtung unverändert beibehält, nachdem der Benutzer in Abhängigkeit von den Hinweisinformationen ausgewählt hat, den Betriebsmodus der elektronischen Vorrichtung nicht auf den vorgegebenen Modus einzustellen, und
das Betriebsmodus-Einstellungsmodul den Betriebsmodus der elektronischen Vorrichtung auf den vorgegebenen Modus einstellt, falls die elektronische Vorrichtung nach Ablauf einer vorgegebenen Zeitspanne, seitdem die elektronische Vorrichtung die Hinweisinformationen angezeigt hat, keine Auswahlanweisung von dem Benutzer empfängt.

4. Elektronische Vorrichtung nach Anspruch 3, ferner aufweisend:
ein Modul zum Senden eines Notsignals, das ein Notsignal aussendet, wenn die Echtzeit-Atemfrequenz des Benutzers um einen ersten vorgegebenen Wert über einer Obergrenze des vorgegebenen Bereichs liegt oder wenn die Echtzeit-Atemfrequenz des Benutzers um einen zweiten vorgegebenen Wert unter einer Untergrenze des vorgegebenen Bereichs liegt.

5. Elektronische Vorrichtung nach Anspruch 3, wobei das Vergleichsmodul ferner konfiguriert ist, um in Abhängigkeit von der Atemfrequenz des Benutzers zu bestimmen, ob der Benutzer gesund ist, und falls bestimmt wird, dass der Benutzer nicht gesund ist, das Vergleichsmodul Hinweisinformationen zur Gesundheitsuntersuchung erzeugt, um den Benutzer an eine Gesundheitsuntersuchung zu erinnern, und die elektronische Vorrichtung die Hinweisinformationen zur Gesundheitsuntersuchung speichert und anzeigt.

6. Elektronische Vorrichtung nach Anspruch 5, ferner aufweisend:
ein Datenspeichermodul, das eine Datenbank mit gesunden Atemfrequenzen speichert,
wobei das Vergleichsmodul die erfasste Atemfrequenz des Benutzers mit den gesunden Atemfrequenzen in der Datenbank vergleicht und bestimmt, dass der Benutzer nicht gesund ist, wenn die Atemfrequenz des Benutzers einen Bereich der gesunden Atemfrequenzen überschreitet, und/oder
die elektronische Vorrichtung ferner aufweist:
ein Kommunikationsmodul, das konfiguriert ist, um einen Bericht der Atemfrequenz des Benutzers an eine Ferndiagnoseplattform zu senden,
wobei die Ferndiagnoseplattform beurteilt, ob der Benutzer gesund ist, und die elektronische Vorrichtung ein von der Ferndiagnoseplattform gesendetes Beurteilungsergebnis empfängt und anzeigt.

7. Elektronische Vorrichtung nach Anspruch 3, ferner aufweisend:
ein benutzerdefiniertes Modul, das konfiguriert ist, um einen vorgegebenen Bereich zu speichern, der der Atemfrequenz des Benutzers entspricht; und/oder
ein Bereichserzeugungsmodul, das konfiguriert ist, um einen durchschnittlichen Atemfrequenzbereich des Benutzers in Abhängigkeit von den Echtzeit-Atemfrequenzen des Benutzers, die von dem Modul zum Erfassen der Echtzeit-Atemfrequenz in einer vorgegebenen Zeitspanne erfasst werden, als den vorgegebenen Bereich zu berechnen.

8. Elektronische Vorrichtung nach Anspruch 3, ferner aufweisend:
ein Geräuscherfassungsmodul, das konfiguriert ist, um ein Echtzeit-Atemgeräusch des Benutzers zu erfassen,
wobei das Modul zum Erfassen der Echtzeit-Atemfrequenz die Echtzeit-Atemfrequenz des Benutzers in Abhängigkeit von dem Echtzeit-Atemgeräusch des Benutzers erfasst, das von dem Geräuscherfassungsmodul erfasst wird.

9. Elektronische Vorrichtung nach Anspruch 3, ferner aufweisend:
einen Atemsensor, der konfiguriert ist, um eine Echtzeit-Atmung des Benutzers zu erfassen, wobei das Modul zum Erfassen der Echtzeit-Atemfrequenz die Echtzeit-Atemfrequenz des Benutzers in Abhängigkeit von der von dem Atemsensor erfassten Echtzeit-Atmung des Benutzers erfasst.

## Revendications

1. Un procédé de commande d'un dispositif électronique, comprenant les étapes suivantes :
acquisition d'une fréquence respiratoire en temps réel d'un utilisateur ;
détermination si la fréquence respiratoire en temps réel de l'utilisateur se situe dans une plage prédéterminée ; et
ajustement sélectif d'un mode de fonctionnement du dispositif électronique vers un mode prédéterminé si la fréquence respiratoire en temps réel de l'utilisateur se situe dans la plage prédéterminée,
dans lequel le mode prédéterminé comprend un mode sans distraction dans lequel une exposition de l'utilisateur à un rayonnement de signaux provenant du dispositif électronique est réduite, et
dans lequel l'étape d'ajustement sélectif du mode de fonctionnement du dispositif électronique vers le mode prédéterminé comprend :
commande du dispositif électronique pour afficher des informations d'invite afin de rappeler à l'utilisateur s'il souhaite ajuster le mode de fonctionnement du dispositif électronique vers le mode prédéterminé, et commande du dispositif électronique pour envoyer un signal de vibration ;
ajustement du mode de fonctionnement du dispositif électronique vers le mode prédéterminé, après que l'utilisateur sélectionne d'ajuster le mode de fonctionnement du dispositif électronique vers le mode prédéterminé conformément aux informations d'invite ;
maintien du mode de fonctionnement du dispositif électronique inchangé, après que l'utilisateur sélectionne de ne pas ajuster le mode de fonctionnement du dispositif électronique vers le mode prédéterminé conformément aux informations d'invite ; et
ajustement du mode de fonctionnement du dispositif électronique vers le mode prédéterminé, si le dispositif électronique ne reçoit pas d'instruction de sélection de la part de l'utilisateur après qu'une période de temps prédéterminée s'est écoulée depuis que le dispositif électronique affiche les informations d'invite.

2. Le procédé de commande selon la revendication 1, dans lequel l'étape d'acquisition de la fréquence respiratoire en temps réel de l'utilisateur comprend :
l'acquisition d'un audio de la respiration en temps réel de l'utilisateur ; et
l'acquisition de la fréquence respiratoire en temps réel de l'utilisateur conformément à l'audio de la respiration en temps réel acquis.

3. Un dispositif électronique, comprenant :
un module d'acquisition de la fréquence respiratoire en temps réel, configuré pour acquérir une fréquence respiratoire en temps réel d'un utilisateur ;
un module de comparaison, configuré pour comparer la fréquence respiratoire en temps réel de l'utilisateur avec une valeur limite supérieure et une valeur limite inférieure d'une plage prédéterminée, et pour générer un résultat de comparaison ;
un module d'ajustement du mode de fonctionnement, configuré pour acquérir le résultat de comparaison généré par le module de comparaison, et pour ajuster sélectivement un mode de fonctionnement du dispositif électronique à un mode prédéterminé si la fréquence respiratoire en temps réel de l'utilisateur est dans la plage prédéterminée ; et
un module de génération d'informations d'invite, qui est capable de générer des informations d'invite rappelant à l'utilisateur s'il souhaite ajuster le mode de fonctionnement du dispositif électronique au mode prédéterminé, si la fréquence respiratoire en temps réel de l'utilisateur est dans la plage prédéterminée,
dans lequel le mode prédéterminé comprend un mode sans distraction dans lequel une exposition de l'utilisateur au rayonnement de signaux provenant du dispositif électronique est réduite, et
dans lequel le dispositif électronique affiche les informations d'invite générées par le module de génération d'informations d'invite, et génère simultanément un signal de vibration,
le module d'ajustement du mode de fonctionnement ajuste le mode de fonctionnement du dispositif électronique au mode prédéterminé, après que l'utilisateur sélectionne d'ajuster le mode de fonctionnement du dispositif électronique au mode prédéterminé conformément aux informations d'invite,
le module d'ajustement du mode de fonctionnement maintient le mode de fonctionnement du dispositif électronique inchangé, après que l'utilisateur sélectionne de ne pas ajuster le mode de fonctionnement du dispositif électronique au mode prédéterminé conformément aux informations d'invite, et
le module d'ajustement du mode de fonctionnement ajuste le mode de fonctionnement du dispositif électronique au mode prédéterminé, si le dispositif électronique ne parvient pas à recevoir une instruction de sélection de l'utilisateur après qu'une période de temps prédéterminée s'est écoulée depuis que le dispositif électronique affiche les informations d'invite.

4. Le dispositif électronique selon la revendication 3, comprenant en outre :
un module d'envoi de signal de détresse, qui envoie un signal de détresse si la fréquence respiratoire en temps réel de l'utilisateur est supérieure à une limite supérieure de la plage prédéterminée d'une première valeur prédéterminée, ou si la fréquence respiratoire en temps réel de l'utilisateur est inférieure à une limite inférieure de la plage prédéterminée d'une seconde valeur prédéterminée.

5. Le dispositif électronique selon la revendication 3, dans lequel le module de comparaison est en outre configuré pour déterminer si un utilisateur est en bonne santé en fonction de la fréquence respiratoire de l'utilisateur, et si l'utilisateur est déterminé comme n'étant pas en bonne santé, le module de comparaison génère des informations d'invite à un contrôle de santé pour rappeler à l'utilisateur d'effectuer un contrôle de santé, et le dispositif électronique stocke et affiche les informations d'invite à un contrôle de santé.

6. Le dispositif électronique selon la revendication 5, comprenant en outre :
un module de stockage de données, qui stocke une base de données de fréquences respiratoires saines,
dans lequel le module de comparaison compare la fréquence respiratoire acquise de l'utilisateur avec des fréquences respiratoires saines dans la base de données, et détermine que l'utilisateur n'est pas en bonne santé si la fréquence respiratoire de l'utilisateur dépasse une plage des fréquences respiratoires saines, et/ou
le dispositif électronique comprenant en outre :
un module de communication, configuré pour envoyer un enregistrement de la fréquence respiratoire de l'utilisateur à une plateforme de diagnostic à distance,
dans lequel la plateforme de diagnostic à distance juge si l'utilisateur est en bonne santé, et le dispositif électronique reçoit et affiche un résultat de jugement envoyé depuis la plateforme de diagnostic à distance.

7. Le dispositif électronique selon la revendication 3, comprenant en outre :
un module défini par l'utilisateur, configuré pour stocker une plage prédéterminée correspondant à la fréquence respiratoire de l'utilisateur ; et/ou
un module de génération de plage, configuré pour calculer une plage de fréquence respiratoire moyenne de l'utilisateur, en tant que plage prédéterminée, en fonction des fréquences respiratoires en temps réel de l'utilisateur acquises par le module d'acquisition de la fréquence respiratoire en temps réel pendant une période de temps prédéterminée.

8. Le dispositif électronique selon la revendication 3, comprenant en outre :
un module de collecte d'audio, configuré pour collecter en temps réel un audio de la respiration de l'utilisateur,
dans lequel le module d'acquisition de la fréquence respiratoire en temps réel acquiert la fréquence respiratoire en temps réel de l'utilisateur conformément à l'audio de la respiration en temps réel de l'utilisateur collecté par le module de collecte d'audio.

9. Le dispositif électronique selon la revendication 3, comprenant en outre :
un capteur de respiration, configuré pour détecter la respiration en temps réel de l'utilisateur,
dans lequel le module d'acquisition de la fréquence respiratoire en temps réel acquiert la fréquence respiratoire en temps réel de l'utilisateur en fonction de la respiration en temps réel de l'utilisateur détectée par le capteur de respiration.
